# EUROPEAN PATENT APPLICATION

(11) **EP 4 328 329 A1**
(43) Date of publication of application: **28.02.2024**
(21) Application number: 22790818.3
(22) Date of filing: 29.03.2022
(51) Int. Cl.: C21B 13/02, C07C 11/04, C07C 11/06

(54) **METHOD FOR PRODUCING SPONGE IRON**

(30) Priority: 23.04.2021 CN 202110440967
(71) Applicant: China University of Petroleum-Beijing, Beijing 102249 (CN); Beijing Carbon Zero Hydrogen Electric Technology Co., Ltd, Beijing 102249 (CN)
(72) Inventor: ZHOU, Hongjun, Beijing 102249 (CN); WU, Quangui, Beijing 102249 (CN); ZHOU, Enze, Beijing 102249 (CN); XING, Xianbo, Beijing 102249 (CN)
(74) Representative: Bergenstråhle & Partners AB
(86) International application number: PCT/CN2022/083725
(87) International publication number: WO 2022/222709

(57) **Abstract**

The present invention provides a method for producing sponge iron comprising the steps of: subjecting a cracking feedstock containing low-carbon alkanes to a steam cracking reaction in which the energy is provided by electricity; separating the products of steam cracking reaction to give a mixed gas containing hydrogen, methane and ethane, as well as ethylene, propylene and/or 1,3-butadiene; and mixing the mixed gas with water and/or CO₂ to produce a syngas for producing sponge iron by a catalytic conversion reaction in which the energy is provided by electricity. The present invention utilizes electricity to provide energy for the steam cracking reaction and the catalytic conversion reaction through an electromagnetic coil, which is a new use of electricity and solve the current problem of excess electricity. Moreover, utilizing the electromagnetic coil to provide power can make the heat distribution in the reaction tube more uniform, and allow easier control of the reaction temperature and the progress of the reaction.

## Description

### Field of Technology

The present invention relates to a method for producing sponge iron and belongs to the technical field of ferrous metallurgy.

### Background Art

Massive carbon dioxide emissions and environmental pollution lead to climate warming and haze, threatening the survival of mankind and the health of the population. An effective solution is to decarbonize energy sources and reduce fossil energy consumption, especially the use of coal, while developing and using renewable energy sources, especially electricity derived from green sources such as photovoltaic, wind and hydropower (referred to as "green electricity").

Unlike fossil energy sources such as coal, oil and natural gas, which can be easily stored, electricity must be produced, transmitted and utilized simultaneously. This feature determines that for electricity as an energy source, the core characteristics of its time dimension must be taken into account and the relationship between the power supply side, electricity transmission side and consumption side must be coordinated with the aim of achieving synchronization. The disconnection of any side will affect the overall situation of electricity.

At present, the production of renewable energy such as photovoltaics, wind power, and hydropower is often spatially mismatched with the consumption side, resulting in a contradiction between the production, transmission and consumption of green electricity. For example, photovoltaic, wind power and hydropower resources are mainly distributed in the northwest and southwest of China, while the energy consumption side is concentrated in the eastern coastal areas, with thousands of kilometers away from each other. The long-distance transmission of electricity is one of the solutions to solve the above problems. Single long-distance output also has the problem of coordination among production, transmission and consumption, and there are problems such as output fluctuation and poor economy, which are difficult to be absorbed. Therefore, a large amount of green electricity generated in the west cannot be exported to the east over long distances, and there are no suitable local consumption scenarios, resulting in large curtailment of wind, water and light power.

Green electricity production must effectively address the two major challenges of local consumption and controllable load fluctuations. The on-site energy storage devices (such as photovoltaic farms with energy storage) can only solve the peak shifting and controllable loads, but it is still difficult to solve the problem of consumption, not to mention that the low-cost storage of large-scale power is hardly realized by the current energy storage technology.

Therefore, to find a new way out for electricity is an urgent technical problem that is needed to be solved in the field of electric power.

### Summary of the invention

In order to solve the above technical problems, an object of the present invention is to provide a method for producing sponge iron. The method utilizes electromagnetic induction to provide energy for a steam cracking reaction on low-carbon alkanes to produce olefins and hydrogen, which are then catalytically converted to obtain syngas for the production of sponge iron. It is capable of replacing the traditional steam cracking energy supply method and provides new prospects for the use of electricity.

In order to achieve the above object, the present invention provides a method for producing sponge iron comprising the following steps of:
subjecting a cracking feedstock containing light alkanes to a steam cracking reaction, wherein the energy for the steam cracking reaction is provided by electricity;
separating the products of the steam cracking reaction to give a mixed gas containing hydrogen, methane and ethane, as well as ethylene, propylene and/or 1,3-butadiene;
mixing the mixed gas containing hydrogen, methane and ethane with water and/or CO₂, and using the resultant as a catalytic conversion feedstock to produce a syngas for producing sponge iron by a catalytic conversion reaction, wherein the energy for the catalytic conversion reaction is provided by electricity.

According to a specific embodiment of the present invention, preferably, the energy is provided by heating a reaction tube for the steam cracking reaction or the catalytic conversion reaction by means of an induction coil, and supplying heat from the reaction tube to the cracking feedstock or the catalytic conversion feedstock. After the induction coil is supplied with power, electromagnetic induction is generated between the reaction tube for the steam cracking reaction or the catalytic conversion reaction and the induction coil, and the reaction tube generates heat, thereby realizing the heating of the cracking feedstock or catalytic conversion feedstock inside the reaction tube. In the process, the induction coil is preferably wrapped around the outside of the reaction tube for the steam cracking reaction or the catalytic conversion reaction, and a thermal insulation material (e.g., cement, fireproof material, etc.) can be filled between the reaction tube and the induction coil. Conventional steam cracking devices and catalytic conversion devices is provided with heat through combustion of fuel oil and gas, and then the reaction tube is heated through heat exchange with the reaction tube to realize the heating of the reaction tube, and thus the cracking feedstock or the catalytic conversion feedstock is heated in the reaction tube. However, such a heat exchange process tends to be not uniform, and the heat would be concentrated in a local area, resulting in that the cracking reaction is conducted non-uniformly. The present invention, on the other hand, heats the reaction tube by means of an induction coil, which has a high heating efficiency, and the induction coil is uniformly distributed on the reaction tube, such that the electromagnetic induction is uniformly generated around the reaction tube, so that uniform heating of the cracking feedstock and the catalytic conversion feedstock is realized.

According to a specific embodiment of the present invention, preferably, the frequency of the current input into the induction coil is medium or high to meet the needs of electromagnetic induction as well as controlling the reaction temperature. In the practice, the frequency of the control current can be selected according to the desired reaction temperature. The high frequency is 5-20 KHz, preferably 8-16 KHz, more preferably 10-15 KHz, further preferably 12-14 KHz, and specifically may be 8 KHz, 8.5 KHz, 9 KHz, 9.5 KHz, 10 KHz, 10.5 KHz, 11 KHz, 11.5 KHz, 12 KHz, 12.5 KHz, 13 KHz, 13.5 KHz, 14 KHz, 14.5 KHz, 15 KHz, 15.5 KHz, 16 KHz, or it may be a range obtained by combining the endpoints of the above ranges as well as the enumerated specific frequency values with each other, such as 5-16 KHz, 5-15 KHz, 5-10 KHz, 8-20 KHz, 8-15 KHz, 8-10 KHz, 10-20 KHz, 10-16 KHz, 10-12 KHz, 9-20 KHz, 9-15 KHz, 12-15 KHz, 12-14 KHz, 12-20 KHz; the medium frequency is 50-3000 Hz, preferably 300-2000 Hz, more preferably 600-1500 Hz, and specifically may be 300 Hz, 400 Hz, 500 Hz, 600 Hz, 700 Hz, 800 Hz, 900 Hz, 1000 Hz, 1100 Hz, 1200 Hz, 1300 Hz, 1400 Hz, 1500 Hz, 1600 Hz, 1700 Hz, 1800 Hz, 1900 Hz, 2000 Hz, or it may be a range obtained by combining the endpoints of the above ranges as well as the enumerated specific frequency values with each other, such as 300-3000 Hz, 300-1500 Hz, 600-3000 Hz, 600-2000 Hz, 1000-3000 Hz, 1000-2000 Hz, 1200-3000 Hz, 1200-2000 Hz, 1500-3000 Hz, 1500-2000 Hz, and the like.

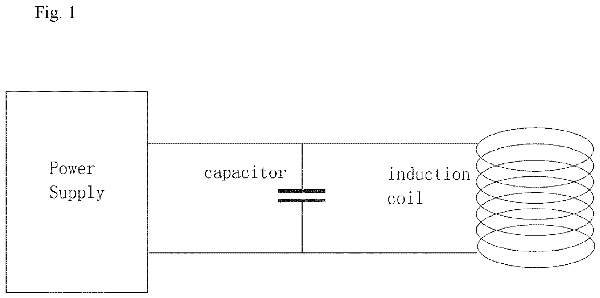

According to a specific embodiment of the present invention, preferably, the frequency of the current input into the induction coil is regulated by a power supply and a capacitor. The induction coil is connected to the power supply to form a circuit, and the power supply is connected in parallel with the capacitor as shown in FIG. 1. The power supply used in the present invention may be a commonly used industrial power supply, such as a medium frequency power supply or a high frequency power supply. The power and other specification parameters of the power supply can be selected according to the frequency that needs to be adjusted to, and the rated power of the power supply is preferably 100-1000 KW, and more preferably 200-500 KW. The specification of the capacitor can also be selected as desired, as long as it is sufficient to be able to be matched with the power supply to meet the frequency control requirements.

According to a specific embodiment of the present invention, preferably, the induction coil is one or a combination of two or more selected from ferrite coils, iron core coils, hollow coils, copper core coils and the like.

According to a specific embodiment of the present invention, preferably, the light alkanes in the cracking feedstock are selected from C2-C8 alkanes; more preferably selected from one or a combination of two or more of ethane, propane, n-butane, iso-butane, n-pentane, iso-pentane, n-hexane, iso-hexane, n-heptane, iso-heptane, n-octane, and iso-octane; further preferably selected from one or a combination of two or more of propane, n-butane, and n-pentane.

According to a specific embodiment of the present invention, preferably, the light alkanes in the cracking feedstock are propane-butane feedstock or n-pentane feedstock. More preferably, the propane-butane feedstock contains propane, n-butane and iso-butane, in an amount of 30-45%, 15-45% and 7-13%, preferably 35-40%, 20-40% and 9-11%, respectively, based on the mass of the propane-butane feedstock.

According to a specific embodiment of the present invention, the reaction temperature of the steam cracking reaction is preferably controlled to be 500-1000°C, more preferably 600-900°C, further preferably 650-850°C, even further preferably 750-850°C or 800-850°C.

According to a specific embodiment of the present invention, preferably, the water-oil ratio for the steam cracking reaction is 0.3-0.7, preferably 0.4-0.5.

According to a specific embodiment of the present invention, the residence time is preferably controlled to be 0.1-1.0 s, more preferably 0.2-0.85 s.

According to a specific embodiment of the present invention, the separation of the products of the steam cracking reaction can be carried out in a conventional manner, as long as it enables ethylene, propylene, 1,3-butadiene to be separated from other gases. After the separation, a mixed gas containing hydrogen, methane and ethane is obtained as well as ethylene, propylene and/or 1,3-butadiene, wherein ethylene, propylene and/or 1,3-butadiene can be exported as a product, and the mixed gas is subjected to a further catalytic conversion, such that the methane and propane and water and/or CO₂ undergo a catalytic conversion to obtain CO and H₂, thereby obtaining a syngas, which is transported to the sponge iron production unit for the production of sponge iron.

According to a specific embodiment of the present invention, preferably, the method further comprises the step of adjusting the composition of the syngas such that a volume percentage content of CO+H₂ is >90%, and a volume ratio of H₂/CO is 1.5-2.5 (preferably 1.7-1.9), so as to be able to enter into a vertical moving bed reactor for the production of sponge iron. In actual production, if the prepared mixed gas contains sulfur, it can be desulfurized in a conventional process before the catalytic conversion.

According to a specific embodiment of the present invention, preferably, the active component for the catalytic conversion reaction is nickel, and the carrier is one or a combination of two or more selected from alumina, magnesium oxide and magnesium aluminate spinel; the active component is included in an amount of 5-20% based on the total mass of the catalyst.

According to a specific embodiment of the present invention, preferably, the catalytic conversion reaction is carried out under the following reaction conditions: a pressure of 0.1-1.0 MPa, a reaction temperature of 500-1100°C (preferably 500-850°C), a space velocity of 500-4,000 h⁻¹ (preferably 500-2000 h⁻¹), and a volume ratio of water and/or CO₂ to CH₄ of 1.2-1.5:1.

According to a specific embodiment of the present invention, the size of the reaction tube for the steam cracking reaction or the catalytic conversion reaction used in the present invention can be selected as desired, wherein the inner diameter of the reaction tube for the steam cracking reaction or the catalytic conversion reaction can be 50-250 mm and the length can be selected according to the reaction requirement.

According to a specific embodiment of the present invention, the material for the reaction tube for the steam cracking reaction or the catalytic conversion reaction may be a metal or an alloy, respectively, including but not limited to the materials typically used for reaction tubes for steam cracking reaction and catalytic conversion reaction. The metal or alloy is preferably one capable of withstanding a temperature of 1000°C, more preferably one capable of withstanding a temperature of 1200°C. The material of the reaction tube for the steam cracking reaction or the catalytic conversion reaction of the present invention may be selected from 316L stainless steel, 304S stainless steel, HK40 high-temperature furnace tube material, HP40 high-temperature furnace tube material, HP Micro Alloy micro-alloyed steel, Manaurite XTM material for steam cracking furnace, or the like.

The traditional olefin industry is a high power-consuming industry, and the traditional ethylene industry consumes about 0.5 tons of fuel per ton of ethylene produced. The famous olefin technology companies in the world include Lummus, S&W, KBR, Linde, TPL/KTI, etc. All steam cracking devices are powered by heating with a steam cracking furnace and a fuel burner tube, of which the structure is complicated, the investment in equipment is large, and the investment in cracking furnace accounts for about 30% of the investment in the whole olefin production. In the present invention, it is changed to electric power supply without burner combustion and flue gas power recovery system, and can realize single stove pipe heating and burning carbon processing, as well as stove pipe internal power supply, which is a feature that is difficult to achieve with conventional combustion heating. Furthermore, it is highly innovative and offers a range of degrees of freedom, significantly simplifying the olefin production process and increasing process flexibility. For the production of olefins and hydrogen by using components of C4 and C5, the equipment investment is small, the structure is simple, and power saving and emission reduction can be realized.

The present invention utilizes electricity to provide power for steam cracking reaction and catalytic conversion reaction through electromagnetic coil, which is a new use of electricity and solve the current problem of excess electricity. Moreover, utilizing the electromagnetic coil to provide power can make the heat distribution of the reaction tube for the steam cracking reaction or the catalytic conversion reaction more uniform, and it is easier to control the reaction temperature and the progress of the reaction.

In the present invention, electricity is applied to the reaction of preparing light olefins by steam cracking of light alkanes and the like, in which the yield of propylene can reach 10% or more; and the yield of ethylene can reach 15% or more, or even close to 40%. Moreover, the conversion of butane is high.

Currently, the hydrogen sources is scarce in iron and steel industry. The source of hydrogen required for ferrous smelting mainly relies on electrolysis of water, but the cost is high and the reaction safety is low. In the technical solution provided by the present invention, electricity is used to supply power for the steam cracking of C4, C5 and the like by means of electromagnetic induction, which is capable of obtaining ethylene and propylene products as well as a mixed-gas containing methane, ethane and hydrogen. The mixed-gas undergoes a further catalytic conversion to obtain syngas, which is used in the production of sponge iron. By using the technical solution of the present invention, a new source of syngas is provided for the iron and steel industry.

### Brief Description of the Drawings

FIG. 1 is a schematic circuit diagram having the power supply, electromagnetic coil, and capacitor in the present invention.

### Detailed Description of Preferred Embodiments

In order to have a clearer understanding of the technical features, objectives and beneficial effects of the present invention, the following detailed description of the technical solutions of the present invention is provided, but it is not to be understood as limiting the implementable scope of the present invention.

### Example 1

This example provides a method for producing sponge iron comprising the following steps:
the feedstock containing butane and pentane is subjected to steam cracking;
the products of steam cracking are separated to give ethylene, propylene and/or 1,3-butadiene, as well as a mixed gas containing hydrogen, methane and ethane;
the mixed gas containing hydrogen, methane and ethane is subjected to the catalytic conversion, wherein the mixed gas is allowed to enter the catalytic conversion reactor tube, and then reacts with the input CO₂ in the catalytic conversion reactor tube to convert hydrocarbons such as methane, ethane, and CO₂ into CO and H₂;
the composition of the catalytically converted gas product is adjusted to the extent that the volume percentage content of CO+H₂ is >90% and the volume ratio of H₂/CO is 1.5-2.5 (preferably 1.7-1.9), and thus it is fed into a gas-based shaft furnace for the production of sponge iron;
in the gas-based shaft furnace, iron ore oxidized pellets are added from the top of the shaft furnace and move from top to bottom, the syngas enters the furnace from the perimeter pipe of the reduction section at the bottom of the shaft furnace and flows from bottom to top, wherein the syngas undergoes a reduction reaction with the oxidized pellets to obtain sponge iron and top gas, with the main reaction: 3H₂+Fe₂O₃=2Fe+3H₂O. There is no carbon dioxide emission during the above process.

The above top gas can be subjected to a washing and cooling treatment, a compression treatment and a desulfurization and decarburization treatment at one time, so as to obtain an unreacted reducing gas (i.e., unreacted blast-furnace gas).

In the above reaction, electricity is used to provide power for the steam cracking reaction and catalytic conversion reaction through electromagnetic induction, which is carried out using the device shown in FIG. 1. The device includes a power supply (300KW medium-frequency power supply), a capacitor (matching with the medium-frequency power supply), an induction coil (copper-core coil, 30cm in length, wrapped around the outside of the reaction tube), a reaction tube for the steam cracking reaction and a reaction tube for the catalytic conversion reaction (316L stainless steel, 30 cm in length, 1.7 cm in inner diameter), wherein the induction coil is connected to the power supply to form a circuit, and the power supply is connected in parallel with the capacitor. The power supply is used to adjust the electricity to a current of appropriate frequency, which is then input into the capacitor, through which the induction coil is powered. Electromagnetic induction generated between the reaction tube for the steam cracking reaction or the catalytic conversion reaction and the energized induction coil starts generating heat, which heats up the steam cracking feedstock and catalytic conversion feedstock inside the reaction tube in order to induce the steam cracking reaction and the catalytic conversion reaction.

In this example, propane, n-butane and n-pentane samples are used as cracking feedstock for the steam cracking reaction, wherein the compositions of the samples are shown in Tables 1-5, respectively.

**Table 1. Composition of propane-butane sample**

| feedstock | ethane | ethylene | propane | propylene | iso-butane | n-butane | trans-buten e | n-butene | iso-butene | cis-butene | n-pentene |
|---|---|---|---|---|---|---|---|---|---|---|---|
| propane-b utane sample | 0.0195 | 0.1055 | 38.9726 | 14.20183 | 9.0246 | 22.5278 | 6.4399 | 4.1753 | 1.6096 | 0.8032 | 0.1026 |

**Table 2. Composition of n-butane sample 1**

| feedstock | ethane | ethylene | propane | propylene | iso-butane | n-butane | trans-butene | n-butene | C5 |
|---|---|---|---|---|---|---|---|---|---|
| n-butane sample 1 | 0.0027 | 0.0135 | 0.031 | 0.0014 | 1.5081 | 98.375 | 0.0029 | 0.0063 | 0.0428 |

**Table 3. Composition of etherified n-butane sample**

| feedstock | n-butane | olefin | propane | C5 | dimethyl ether | iso-butane |
|---|---|---|---|---|---|---|
| etherified n-butane | 49.2428 | 3.3244 | 0.0152 | 0.0460 | 0.0158 | 47.3296 |

**Table 4. Composition of n-butane sample 2**

| feedstock | methane | ethane | ethylene | propane | propylen e | iso-butan e | n-butane | trans-but ene | n-butene | iso-buten e | cis-buten e | iso-penta ne | n-pentan e | butadien e |
|---|---|---|---|---|---|---|---|---|---|---|---|---|---|---|
| n-butane sample 2 | 0.0034 | 0.0035 | 0.0213 | 0.001 | 0.0188 | 1.2194 | 98.3186 | 0.0016 | 0.001 | 0.0064 | 0.0056 | 0.0044 | 0.0119 | 0.0247 |

**Table 5. Composition of n-pentane sample**

| feedstock | propane | propylene | iso-butane | n-/iso-butene | n-butane | C5 |
|---|---|---|---|---|---|---|
| YATONG n-pentane sample | 0.05 | 0.62 | 10.26 | 0.33 | 35.28 | 53.46 |

The reaction conditions and reaction results are shown in Table 6. In Table 6, in addition to methane, ethane, ethylene, propane, propylene, butane, and pentane, the remaining component in the steam cracking products is mainly hydrogen. In Table 6, "/" represents that the corresponding data were not detected, e.g., n-pentane samples were not detected for the conversions of propane and butane.

From Table 6, it can be seen that electrical energy is applied to the reaction for preparing light olefins by steam cracking of light alkanes and the like, the yield of propylene can reach 10% or more; and the yield of ethylene can reach 15% or more, or even close to 40%. Moreover, the conversion of butane is high.

The voltage, current and power given in Table 6 are parameters under experimental conditions. In industrial applications, for example, the reaction tube would have a larger size, and the degree of reaction will be different from the experimental conditions. Industrial electricity is generally 220V three-phase or 380V three-phase power, and the current and power can be adjusted according to the actual situation (Table 7 shows the upper limit of the parameters under industrial electricity conditions). This difference in parameters does not make a substantial difference to the products.

For the catalytic conversion, as an example, the specific process for a product with 59.861% hydrogen and 19.962% methane is as follows:
an appropriate amount of CO₂ is introduced to adjust the volume ratio of methane, CO₂ and H₂ to 20:20:60 in the feed gas;
by using MC6-0406S as a catalyst, the reaction temperature is started from 500°C and gradually increased to 850°C with a space velocity of 2000 h⁻¹ and a pressure of 0.5 MPa; the corresponding catalytic conversion results are shown in Table 8;
after the catalytic conversion is completed, an appropriate amount of gas can be added to make the syngas meet the following requirements: the volume percentage content of CO+H₂ is >90%, and the volume ratio of H₂/CO is 1.5-2.5 (preferably 1.7-1.9).

The syngas that meets the requirements can be fed into a gas-based shaft furnace for the production of sponge iron.

**Table 6**

| No. | feedsto ck | Reaction Conditions | | | | | | | Products | | | | | | | |
|---|---|---|---|---|---|---|---|---|---|---|---|---|---|---|---|---|
| | | W/0 ratio | Vol. | Cur. | Pow | Fre. | Temp. | Time | methane | ethane | ethylen e | propane | propy le ne | butadi ene | propane conversio n | butane conversion |
| | | | V | A | KW | KHZ | °C | s | | | | | | | | |
| 1 | propane -butane sample | 0.4 | 40 | 8.9 | 0.5 | 13.3 | 650 | 0.85 | 1.3356 | 9.3497 | 1.1766 | 46.6707 | 3.2406 | / | 4.93% | / |
| 2 | | | 40 | 8.9 | 0.5 | 13.3 | | | 1.0278 | 8.9733 | 0.9592 | 47.0206 | 2.9325 | / | 4.22% | / |
| 3 | | | 37 | 8.7 | 0.4 | 12.7 | 750 | | 5.9822 | 8.5467 | 9.4662 | 35.3118 | 12.4083 | / | 28.07% | 23.52% |
| 4 | | | 37 | 8.7 | 0.4 | 12.7 | | | 6.4898 | 8.4439 | 10.2958 | 34.0575 | 13.3391 | / | 30.62% | 26.85% |
| 5 | | | 37 | 8.9 | 0.5 | 12.6 | 850 | | 19.962 | 6.855 | 35.693 | 11.4926 | 15.1148 | / | 76.59% | / |
| 6 | | | 37 | 8.9 | 0.5 | 12.6 | | | 30.1648 | 7.792 | 39.1184 | 7.1282 | 10.4303 | / | 85.48% | 90.09% |
| 7 | propane -butane sample | 0.4 | 42 | 9.2 | 0.6 | 13.9 | 650 | 0.3 | 0.2056 | 6.5955 | 0.415 | 47.2838 | 2.2069 | / | 4.09% | / |
| 8 | | | 42 | 9.2 | 0.6 | 13.9 | | | 0.191 | 6.253 | 0.409 | 47.2532 | 2.2287 | / | 4.15% | / |
| 9 | | | 43 | 9.5 | 0.6 | 14.2 | 750 | | 1 .7592 | 5.8202 | 2.9659 | 43.4773 | 5.695 | / | 11.81% | 2.24% |
| 10 | | | 43 | 9.5 | 0.6 | 14.2 | | | 1 .6273 | 5.6007 | 2.809 | 43.6184 | 5.4855 | / | 11 .5 3% | 0.76% |
| 11 | | | 37 | 8.9 | 0.5 | 12.6 | 850 | | 9.6641 | 4.923 | 16.966 | 28.9745 | 11.6946 | / | 41.23% | 36.31% |
| 12 | | | 37 | 8.9 | 0.5 | 12.6 | | | 9.1522 | 4.693 1 | 15.9151 | 28.7863 | 11.476 | / | 41.61% | 36.03% |
| 13 | n-butane sample 1 | 0.4 | 43 | 9.8 | 0.6 | 14 | 800 | 0.2 | 5.0814 | 1.122 | 11.4951 | 0.1698 | 8.1726 | / | / | 27.46% |
| 14 | | | 43 | 9.8 | 0.6 | 14 | | | 5.0412 | 1.2329 | 10.8214 | 0.1722 | 8.2897 | / | / | 27.16% |
| 15 | | | 49 | 10.5 | 0.8 | 14.7 | 830 | | 7.2632 | 1.6196 | 15.3564 | 0.2038 | 8.8526 | / | / | 35.22% |
| 16 | | | 49 | 10.6 | 0.8 | 14.8 | | | 6.3326 | 1.3903 | 13.9567 | 0.1874 | 8.0361 | / | / | 31.70% |
| 17 | n-butane sample 1 | 0.4 | 39 | 9 | 0.5 | 13.2 | 800 | 0.2 | 7.8267 | 2.0546 | 14.2414 | 0.2279 | 10.325 | / | / | 36.44% |
| 18 | | | 39 | 9 | 0.5 | 13.2 | | | 6.9674 | 1.8023 | 12.8967 | 0.2071 | 9.0487 | / | / | 32.49% |
| 19 | | | 49 | 10.8 | 0.8 | 14.9 | 830 | | 11.801 | 2.6253 | 20.1379 | 0.2664 | 9.5428 | / | / | 46.09% |
| 20 | | | 49 | 10.8 | 0.8 | 14.9 | | | 9.9053 | 2.1707 | 18.3217 | 0.2346 | 8.6324 | / | / | 40.89% |
| 21 | ether if ied n-but ane sample | 0.4 | 39 | 8.8 | 0.5 | 13.2 | 650 | 0.3 | 0.2182 | 0.061 | 0.2116 | 0.0027 | 0.6061 | / | / | 1.30% |
| 22 | | | 39 | 8.8 | 0.5 | 13.2 | | | 0.3231 | 0.0857 | 0.3327 | 0.005 | 0.9123 | / | / | 1.97% |
| 23 | | | 42 | 9.4 | 0.6 | 13.8 | 750 | | 3.04 | 0.5748 | 4.0627 | 0.0739 | 6.9343 | / | / | 17.17% |
| 24 | | | 42 | 9.4 | 0.6 | 13.8 | | | 3.0875 | 0.6108 | 3.9479 | 0.079 | 6.8393 | / | / | 17.21% |
| 25 | | | 42 | 9.4 | 0.6 | 13.8 | | | 20.9053 | 3.3027 | 19.0408 | 0.3162 | 11.5655 | / | / | 59.20% |
| 26 | | | 43 | 9.7 | 0.6 | 13.9 | 800 | | 20.23 16 | 3.1882 | 20.42 | 0.3034 | 11.5117 | / | / | 59.56% |
| 27 | | | 43 | 9.7 | 0.6 | 13.9 | | | 9.9083 | 1.668 | 10.853 | 0.2229 | 9.3724 | / | / | 35.17% |
| 28 | | | 43 | 9.7 | 0.6 | 13.9 | | | 11.7654 | 1.9387 | 12.1556 | 0.2347 | 10.1396 | / | / | 39.66% |
| 24 | n-butane sample 2 | 0.4 | 66.01 | 6.27 | 1.09 | 13.2 | 650 | 0.3 | 2.353 | 0.6589 | 3.9915 | 0.0548 | 5.4968 | / | / | 12.76% |
| 30 | | | 75.62 | 9.66 | 1.24 | 13.1 | | | 2.4208 | 0.7515 | 4.2058 | 0.0666 | 5.8335 | / | / | 13.69% |
| 31 | | | 77 | 9.77 | 1.24 | 14.0 | 750 | | 7.2971 | 1.8985 | 13.962 | 0.2 18 | 12.2415 | / | / | 37.63% |
| 32 | | | 75.62 | 9.27 | 1.24 | 13.9 | | | 8.2876 | 2.0795 | 16.1294 | 0.2478 | 13.5253 | / | / | 42.64% |
| 33 | n-butane sample 2 | 0.4 | 82.5 | 10.29 | 1.2 | 14.8 | 850 | 0.3 | 23.0038 | 3.9408 | 34.4269 | 0.3149 | 9.9142 | / | / | 70.84% |
| 34 | | | 104.49 | 12.94 | 1.39 | 14.6 | | | 23.7909 | 4.0855 | 38.1567 | 0.3351 | 10.3486 | | / | 77.64% |
| 35 | n-pentane sample | 0.4 | 15.11 | 1.27 | 0.79 | 13.5 | 800 | 0.7 | 20.587 | 5.1823 | 2.9.3074 | 0.6159 | 16.1166 | 2.2276 | / | / |
| 36 | | | 77 | 9.77 | 1.2 | 13.8 | | | 30.1312 | 6.0186 | 37.6199 | 0.3935 | 9.6449 | 2.4089 | / | / |
| 37 | | | 79.77 | 10.01 | 1.16 | 14.1 | 850 | | 37.2192 | 3.8467 | 43.1129 | 0.1852 | 5.2999 | 2.8257 | / | / |
| 38 | | | 77, | 9.95 | 1.16 | 14.2 | | | 34.1595 | 5.0135 | 42.8161 | 0.2805 | 7.268 | 2.9477 | / | / |

**[Table 7. Upper limits of parameters under industrial electricity conditions]**

| | | | |
|---|---|---|---|
| Power | Voltage | Current | Frequency |
| 200KW | three-phase 380V | 305A | 5-20KHz |
| 300KW | three-phase 380V | 455A | 5-20KHz |
| 500KW | three-phase 380V | 760A | 5-20KHz |
| 200KW | three-phase 220V | 530A | 5-20KHz |
| 300KW | three-phase 220V | 790A | 5-20KHz |
| 500KW | three-phase 220V | 1320A | 5-20KHz |

**Table 8**

| H₂ | CH₄ | CO₂ | CO | Reaction temperatur e/°C | Conversio n of CH₄ | Total conversion of CH₄ | Conversio n of CO₂ |
|---|---|---|---|---|---|---|---|
| 59.861 | 19.962 | 20.177 | 0.000 | | | | |
| 28.868 | 54.124 | 15.176 | 1.833 | 501 | 2.60% | -51.71% | 57.56% |
| 31.890 | 50.205 | 13.827 | 4.079 | 601 | 6.76% | -66.71% | 54.62% |
| 46.829 | 30.683 | 10.319 | 12.170 | 701 | 28.60% | -43.55% | 52.19% |
| 56.669 | 18.517 | 7.396 | 17.419 | 802 | 47.10% | -0.15% | 60.46% |
| 59.321 | 16.296 | 7.173 | 17.221 | 852 | 46.30% | 12.32% | 61.82% |
| 60.157 | 15.214 | 6.598 | 18.032 | 850 | 49.12% | 17.12% | 64.44% |

## Claims

1. A method for producing sponge iron, comprising the steps of:
subjecting a cracking feedstock containing light alkanes to a steam cracking reaction, wherein the energy for the steam cracking reaction is provided by electricity;
separating the products of the steam cracking reaction to give a mixed gas containing hydrogen, methane and ethane, as well as ethylene, propylene and/or 1,3-butadiene;
mixing the mixed gas containing hydrogen, methane and ethane with water and/or CO₂, and using the resultant as a catalytic conversion feedstock to produce a syngas for producing sponge iron, wherein the energy for the catalytic conversion reaction is provided by electricity.

2. The method according to claim 1, wherein the energy is provided by heating a reaction tube for the steam cracking reaction or the catalytic conversion reaction by means of an induction coil, and supplying heat from the reaction tube to the cracking feedstock or the catalytic conversion feedstock.

3. The method according to claim 2, wherein the induction coil is wrapped around the outside of the reaction tube.

4. The method according to claim 2, wherein the current input into the induction coil has a medium frequency or a high frequency.

5. The method according to claim 4, wherein the high frequency is 5-20 KHz.

6. The method according to claim 5, wherein the high frequency is 8-16 KHz.

7. The method according to claim 6, wherein the high frequency is 10-15 KHz.

8. The method according to claim 4, wherein the medium frequency is 50-3000 Hz.

9. The method according to claim 8, wherein the medium frequency is 300-2000 Hz.

10. The method according to claim 2, wherein the frequency of the current input to the induction coil is regulated by a power supply and a capacitor.

11. The method according to claim 10, wherein the induction coil is connected to the power supply to form a circuit, and the power supply is connected in parallel with the capacitor.

12. The method according to claim 10, wherein the power supply has a power of 100-1000 KW.

13. The method according to claim 12, wherein the power supply has a power of 200-500 KW.

14. The method according to claim 2, wherein the induction coil is one or a combination of two or more of a ferrite coil, an iron core coil, a hollow coil, and a copper core coil.

15. The method according to claim 1, wherein the light alkanes in the cracking feedstock are selected from C2-C8 alkanes.

16. The method according to claim 15, wherein the light alkanes in the cracking feedstock are selected from one or a combination of two or more of ethane, propane, n-butane, iso-butane, n-pentane, iso-pentane, n-hexane, iso-hexane, n-heptane, iso-heptane, n-octane, and iso-octane.

17. The method according to claim 16, wherein the light alkanes in the cracking feedstock are selected from propane, n-butane, and n-pentane.

18. The method according to claim 1 or 3, wherein the light alkanes in the cracking feedstock are propane-butane feedstock or n-pentane feedstock.

19. The method according to claim 18, wherein the propane-butane feedstock contains propane, n-butane and iso-butane in an amount of 30-45%, 15-45% and 7-13%, respectively, based on the mass of the propane-butane feedstock.

20. The method according to claim 19, wherein the propane, n-butane and iso-butane are included in amount of 35-40%, 20-40% and 9-11%, respectively, based on the mass of the propane-butane feedstock.

21. The method according to claim 1, wherein the steam cracking reaction is carried out at a reaction temperature of 500-1000°C.

22. The method according to claim 21, wherein the steam cracking reaction is carried out at a reaction temperature of 600-900 °C.

23. The method according to claim 22, wherein the steam cracking reaction is carried out at a reaction temperature of 650-850 °C.

24. The method according to claim 23, wherein the steam cracking reaction is carried out at a reaction temperature of 800-850 °C.

25. The method according to claim 1, wherein the water-oil ratio for the steam cracking reaction is 0.3-0.7.

26. The method according to claim 25, wherein the water-oil ratio for the steam cracking reaction is 0.4-0.5.

27. The method according to claim 1, wherein the residence time is 0.1-1.0 s.

28. The method according to claim 27, wherein the residence time is 0.2-0.85 s.

29. The method according to claim 1, further comprising a step of adjusting the composition of the syngas such that a volume percentage content of CO+H₂ is >90%, and a volume ratio of H₂/CO is 1.5-2.5.

30. The method according to claim 29, wherein the volume ratio of H₂/CO is 1.7-1.9.

31. The method according to claim 1, wherein the catalyst for the catalytic conversion reaction has an active component of nickel, and a carrier which is one or a combination of two or more selected from alumina, magnesium oxide and magnesium aluminate spinel; and the active component is included in an amount of 5-20% based on the total mass of the catalyst; and
the catalytic conversion reaction is carried out under the following reaction conditions: a pressure of 0.1-1.0 MPa, a reaction temperature of 500-1,100°C, a space velocity of 500-4,000 h⁻¹, and a volume ratio of water and/or CO₂ to CH₄ of 1.2-1.5:1.

32. The method according to claim 31, wherein the reaction temperature is 500-850°C.

33. The method according to claim 31, wherein the space velocity is 500-2,000h⁻¹.

34. The method according to claim 2, wherein the material for the reaction tube for the steam cracking reaction or the catalytic conversion reaction is a metal or an alloy.

35. The method according to claim 34, wherein the metal or alloy is one capable of withstanding a temperature of 1,000°C.

36. The method according to claim 35, wherein the metal or alloy is one capable of withstanding a temperature of 1,200°C.

37. The method according to claim 36, wherein the metal or alloy is selected from 316L stainless steel, 304S stainless steel, HK40 high-temperature furnace tube material, HP40 high-temperature furnace tube material, HP Micro Alloy micro-alloy steel or Manaurite XTM material for steam cracking furnace.

38. The method according to claim 34, wherein the reaction tube for the steam cracking reaction or the catalytic conversion reaction has an inner diameter of 50-250 mm.
